Europäisches Patentamt

European Patent Office

Office européen des brevets

(19)

(11) **EP 0 467 762 B1**

(12) **FASCICULE DE BREVET EUROPEEN**

(45) Date de publication et mention
de la délivrance du brevet:
**09.10.1996 Bulletin 1996/41**

(51) Int Cl.[6]: **C07H 13/04**

(21) Numéro de dépôt: **91401946.8**

(22) Date de dépôt: **12.07.1991**

(54) **Procédé de préparation de monomères de saccharides comportant au moins un groupement carbonyloxyvinylique polymérisable**

Verfahren zur Herstellung von mindestens eine polymerisierbare Carbonyloxyvinylgruppe enthaltenden Saccharid-Monomeren

Method for the preparation of saccharide monomers containing at least one polymerisable vinylic carbonyloxy group

(84) Etats contractants désignés:
**AT BE CH DE DK ES FR GB GR IT LI LU NL SE**

(30) Priorité: **16.07.1990 FR 9009015**

(43) Date de publication de la demande:
**22.01.1992 Bulletin 1992/04**

(73) Titulaire: **ERIDANIA BEGHIN-SAY**
**F-59239 Thumeries (FR)**

(72) Inventeurs:
• **Mentech, Julio**
**F-69100 Villeurbanne (FR)**
• **Betremieux, Isabelle**
**F-69100 Villeurbanne (FR)**
• **Leger, Bruno**
**F-69100 Villeurbanne (FR)**

(74) Mandataire: **David, Daniel et al**
**KAYSERSBERG,**
**Service Propriété Industrielle,**
**23 boulevard Georges Clemenceau**
**92402 Courbevoie Cédex (FR)**

(56) Documents cités:
**DE-A- 2 940 911**       **DE-A- 2 940 952**
**US-A- 4 833 202**

• **CHEMICAL ABSTRACTS, vol. 87, no. 18, 31 octobre 1977, page 87, abrégé no.**
• **137594n, Columbus, Ohio, US; & SU-A-567 726 (INSTITUTE OF WOOD PULP CHEMISTRY, ACADEMY OF SCIENCES, LATVIAN S. S.R.) 05-08-1977**
• **JOURNAL OF THE AMERICAN CHEMICAL SOCIETY, vol. 72, no. 3, 23 mars 1950, pages 1137-1140; M. ZIEF: "Unsaturated esters of sucrose"**
• **MONATSHEFTE FÜR CHEMIE, vol. 112, 1981, pages 273-285, Springer Verlag; H. GRUBER: "Hydrophile Polymergele mit reaktiven Gruppen, 1. Mitt.: Herstellung und Polymerisation von Glucose- und Saccharosemethacrylaten"**
• **DIE MAKROMOLEKULARE CHEMIE, vol. 117, no. 2817, 1968, pages 210-214; W.A.P. BLACK et al.: "6-O-methacryloyl-D-galactose: A reactive, water-soluble monomer"**

## Description

La présente invention concerne un nouveau procédé de préparation de monomères de saccharides comportant au moins un groupement acrylate ou méthylacrylate polymérisable. Plus particulièrement l'invention se rapporte à un procédé de préparation direct et contrôlé d'esters de type acrylique de saccharides choisis parmi les mono- di - et trisaccharides, en particulier les mono -, di et trisaccharides non réducteurs, dans un milieu réactionnel aqueux.

Les monomères de saccharides comportant au moins un groupement acrylate ou méthylacrylate polymérisable sont principalement utilisés dans le domaine des polymères après homo-ou copolymérisation avec divers comonomères.

Diverses méthodes de préparation d'esters de type acrylique de saccharides ont déjà été mises en oeuvre mais aucune d'elles ne s'est avérée satisfaisante.

En effet, les méthodes nécessitent dans la plupart des cas des étapes préliminaires de protection de certaines fonctions réactives de la molécule du saccharide de départ afin de permettre la modification sélective des fonctions résiduelles que l'on souhaite estérifier. Les étapes intermédiaires entraînent des synthèses longues et coûteuses qu'il est souhaitable d'éviter. De telles réactions sont décrites notamment dans les publications suivantes : T.P. Bird et al, J. Chem. Soc., 1966, 1913-18 (1) ; W.A.P. Black et al, Carbohyd. Res., 5 (1967), 362-365 (2) ; W.A.P. Black et al, Makromol. Chem., 117 (1968), 210 (3) ; W.A.P. Black et al, J. Chem. Soc., 1963, 4433 (4). Par ailleurs, les brevets EP 180262, EP 237131, EP 237132 et US 4721760 de Shell Oil Company décrivent la synthèse de polymères solubles dans l'eau, obtenus par polymérisation d'un monomère de saccharide, le 3-0-méthacrylate de glucose, synthétisé selon des méthodes décrites dans les publications précitées. Le document "Monatshefte für Chemie, vol. 112, 1981, pages 273-285" décrit des monomères de glucose contenant le groupement méthacryloyle. On connaît d'autres méthodes de préparation d'esters de type acrylique de saccharides utilisant des approches plus directes. Ces méthodes sont décrites notamment dans les publications suivantes : E. Avela et al "Sucrochemistry", Washington, American Chemical Society 1977, S, 62 (ACS Symposium Series N° 41) ; H. Grüber, Monatsch. Chem. 1981, 112, 273-85. Cependant de telles réactions mettent en jeu des solvants de type aprotique polaire (DMF, DMSO, pyridine, etc.) qui conduisent à des problèmes supplémentaires liés à leur coût, toxicité, inflammabilité, etc. ...

Il est en outre nécessaire, lors de l'utilisation en tant que monomères et comonomères des esters de type acrylique des saccharides choisis, d'obtenir un excellent contrôle du degré de substitution de la molécule de substrat. En effet, une valeur moyenne du degré de substitution du monomère en question supérieure à environ 1 entraînera lors de sa polymérisation un certain taux de réticulation. Le taux de réticulation détermine à son tour les diverses propriétés (physiques, chimiques, etc.) des polymères obtenus.

Par ailleurs, il peut être souhaitable, par exemple dans le cas où l'on désire obtenir des copolymères thermoplastiques à partir des monomères en question, qu'ils soient linéaires, autrement dit que leur taux de réticulation soit nul. Pour la fabrication de tels polymères linéaires, il est donc nécessaire de disposer de monomères esters de type acrylique de saccharides ayant un degré de substitution très voisin de 1 et donc de disposer d'un procédé de préparation d'esters de type acrylique de saccharides permettant de contrôler leur degré de substitution. Un tel contrôle a été obtenu en utilisant les méthodes de préparation indirectes précitées mais ces dernières sont inexploitables industriellement du fait de leurs nombreux inconvénients susmentionnés.

De plus, les méthodes directes signalées plus haut ne permettent pas un contrôle satisfaisant du degré de substitution et, de toute façon, l'emploi des solvants organiques qu'elles nécessitent les rendent pratiquement inexploitables d'un point de vue commercial.

Un objet de la présente invention est de disposer d'un procédé de préparation de monomères comportant au moins un groupement acrylate ou méthylacrylate polymérisable, c'est-à-dire d'esters de type acrylique de saccharides en une seule étape ne nécessitant pas l'emploi de solvants organiques.

Un objet de l'invention est aussi de préparer des esters de type acrylique de saccharides tout en obtenant en outre un excellent contrôle de leur degré de substitution.

Un autre objet de l'invention est d'obtenir des esters de type acrylique de saccharides ayant un degré de substitution sensiblement égal à 1.

La présente invention fournit un procédé de préparation de monomères comportant au moins un groupement acrylate ou méthylacrylate polymérisable, en particulier d'esters de type acrylique de saccharides répondant aux objets ci-dessus.

Selon la présente invention, il est donc fourni un procédé de préparation de monomères comportant au moins un groupement acrylate ou méthylacrylate polymérisable, en particulier d'esters de type acrylique de saccharide consistant à faire réagir en milieu aqueux ledit saccharide avec un réactif estérifiant, en présence d'une base, en maintenant le pH à une valeur comprise dans l'intervalle de 7 à 11 pendant toute la durée de la réaction.

Ledit saccharide de départ est choisi parmi les mono-, di- et trisaccharides, en particulier les mono-, di- et trisaccharides non réducteurs. Un exemple d'un tel saccharide est le saccharose.

Ledit réactif estérifiant est choisi parmi les halogénures, par exemple les chlorures d'acryloyle et de méthylacryloyle, les alkylesters acryliques ou méthacryliques, le radical alkyl ayant de 1 à 3 atomes de carbone, tels l'acrylate de méthyl ou le méthylacrylate de méthyl, et les anhydrides d'acides correspondants par exemple l'anhydride acrylique ou méthacrylique.

Ladite base peut être de nature organique ou, de préférence, minérale. Parmi les bases utilisables selon l'invention on peut citer de façon non limitative Na$_2$CO$_3$, NaOH, KOH.

La valeur du pH du milieu réactionnel doit être maintenue pendant toute la durée de la réaction dans l'intervalle de 7 à 11, de préférence entre 10 et 11.

La durée de réaction sera aisément déterminée par l'homme de l'art en fonction notamment du degré de substitution souhaité en tenant compte des valeurs des autres paramètres réactionnels. On veillera également à éviter des temps de réaction trop longs qui peuvent favoriser les réactions inverses d'hydrolyse des esters formés.

La réaction peut être effectuée sous pression atmosphérique à une température de 0°C à environ 60°C, de préférence de 0°C à environ 30°C.

Le rapport molaire entre ledit réactif estérifiant et ledit saccharide de départ est un paramètre réactionnel important. Pour l'obtention de produits monosubstitués le rapport molaire réactif estérifiant sur saccharide sera compris entre environ 0,1 et 8, de préférence entre environ 0,2 et 2, mieux encore entre environ 0,2 et 1,5. Pour l'obtention de produits plus substitués la valeur dudit rapport molaire peut être supérieure à 8.

La stabilisation des réactifs et/ou des produits lors de leur préparation ou pendant la phase éventuelle ultérieure de purification peut s'avérer nécessaire. Une telle stabilisation peut par exemple être effectuée par ajout d'un inhibiteur de polymérisation de type classique tel le 2,6-di-tert-butyl-4-méthylphénol commercialisé sous la marque "Ionol" par la société Shell Chemical Co., la benzoquinone, etc..

Ainsi que précédemment mentionné et comme cela sera illustré plus en details dans les Exemples ci-après, selon les conditions choisies, la teneur en produits monosubstitués ou polysubstitués sera majoritaire.

Le procédé de la présente invention permet d'obtenir de façon controlée aussi bien des produits ayant un degré de substitution sensiblement égal à 1 que des produits ayant un degré de substitution supérieur à 1.

Il est souhaitable, pour certaines applications, de diminuer au maximum le taux de produits polysubstitués des monomères afin, par exemple, de pouvoir préparer des polymères linéaires. Il est alors approprie de procéder à une phase de purification. Les méthodes de purification classiques connues peuvent être appliquées à cet effet, en particulier la chromatographie ou la cristallisation fractionnée, ou, de façon préférée, l'extraction liquide-liquide.

En procédant par extraction liquide-liquide, on extrait dans un premier temps du milieu réactionnel aqueux les dérivés polysubstitués, qui se sont formés en même temps que les dérivés monosubstitués recherchés, par un solvant organique ou un mélange de solvants organiques, comme par exemple la méthyléthyl cétone, le 2-butanol, etc.

Dans un deuxième temps, on met en oeuvre une autre combinaison de solvants d'extraction, en général plus polaire, comme par exemple le n-butanol, le 2-butanol, l'isopropanol, les mélanges éthanol-méthanol, qui permet l'extraction des produits monosubstitués recherchés.

Au cours de cette deuxième étape de purification une certaine quantité de saccharide de départ non-réagi est extraite avec le dérivé monomère monosubstitué recherché, ce qui n'a pas de conséquence pour la polymérisation ultérieure du monomère du fait de la nature inerte dudit saccharide libre en polymérisation.

Les phases organiques contenant les produits souhaités sont stabilisées avec un inhibiteur de polymérisation adéquat et évaporées sous pression réduite pour conduire à des produits solides secs.

Avant de polymériser les monomères fabriqués, il est souhaitable de laver le produit avec un solvant organique, tel l'hexane, l'éther, CH$_2$Cl$_2$, etc., .., pour éliminer l'inhibiteur précédemment ajouté (l'Ionol™).

Le procédé de la présente invention permet de fabriquer, en milieu aqueux, des monomères de saccharides comportant au moins un groupement acrylate ou méthylacrylate polymérisable avec un excellent contrôle de leur degré de substitution et peut conduire en outre à l'obtention de tels monomères acrylate ou méthylacrylate de saccharides ayant un degré de substitution sensiblement égal à 1, c'est-à-dire compris entre 1 et environ 1,1.

Les Exemples suivants illustrent l'invention sans la limiter.

Exemple 1:

On a introduit dans un réacteur de 250 ml 25 g de saccharose (73 mM) dans 75 ml d'eau. On a amené le pH de la solution à 10,5 à l'aide de soude 6N et on l'a maintenu à cette valeur pendant toute la réaction. On a coulé goutte à goutte 3 ml de chlorure d'acryloyle (36,5 mM) sous agitation à température ambiante. Après 15 mn on a neutralisé le milieu réactionnel

La composition du mélange réactionnel brut en produits organiques d'après analyse chromatographique sur couche mince est la suivante :

- saccharose non réagi : 60 %
- monoacrylates de saccharose : 20 %
- polyacrylates de saccharose : 20 %

La répartition molaire de la fraction purifiée (12 g) en monoacrylates de saccharoses après plusieurs extractions, d'après analyse HPLC est la suivante :

- saccharose : 53 %
- monoacrylates de saccharose : 47 %

Exemple 2 :

On a introduit dans un réacteur de 250 ml 25 g de saccharose (73 mM) dans 75 ml d'eau. On a amené le pH de la solution à 10,5 à l'aide de soude 6N et on a maintenu à cette valeur pendant toute la réaction. On a coulé goutte à goutte 8 ml de chlorure de méthacryloyle (73 mM) sous agitation à température ambiante. A la fin de la coulée on a neutralisé le milieu réactionnel.

Le taux de transformation du saccharose (T.T.) et la composition du mélange réactionnel brut en produits organiques déterminés d'après les analyses C.C.M. et HPLC sont les suivants :

- T.T. (saccharose) : 54 %
- saccharose non réagi : 40 %
- monométhacrylates de saccharose : 40 %
- polyméthacrylates de saccharose : 20 %

Exemple 3 :

On a répété l'Exemple 2 ci-dessus avec les quantités suivantes :

- saccharose : 25 g (73 mM)
- chlorure de méthacryloyle : 4 ml (36,5 mM)
- soude 6N

dans 75 ml d'eau.

On a obtenu les résultats suivants :

- T.T. (saccharose) : 35 %
- saccharose non réagi : 50 %
- monométhacrylates de saccharose : 35 %
- polyméthacrylates de saccharose : 15 %

Exemple 4 :

On a répété l'Exemple 2 ci-dessus avec les quantités suivantes :

- saccharose : 250 g (730 mM)
- chlorure de méthacryloyle : 2 ml (180 mM)
- soude 6N

dans 750 ml d'eau.

On a obtenu les résultats suivants :

- T.T. (saccharose) : 24 %
- saccharose non réagi : 70 %
- monométhacrylate de saccharose : 25 %
- polyméthacrylates de saccharose : 5 %

Exemple 5 : Purification des monométhacrylates de saccharose par extraction liquide-liquide

On a saturé le mélange réactionnel brut obtenu dans l'Exemple 4 ci-dessus en chlorure de sodium et on l'a extrait avec des mélanges de méthyl-éthyl cétone et de butanol-2 (50/50 ; 2 x 0.5 l) pour extraire les polyméthacrylates, puis avec du butanol-2 (6 x 0,5 l) pour obtenir, après évaporation du butanol-2 en présence d'environ 2 % en poids 2,6-di-ter-butyl-4-méthylphénol (Ionol) comme stabilisant des monométhacrylates, un mélange (75 g) contenant des monométhacrylates de saccharose (80 %) et du saccharose (20 %), sans polyméthacrylates, et enrichi en monométhacrylates par rapport au mélange réactionnel brut traité.

L'Ionol utilisé pour stabiliser la fraction de monométhacrylates peut être enlevé avant leur polymérisation en effectuant plusieurs lavages au dichlorométhane.

Exemple 6 : Purification des monométhacrylates de saccharose par chromatographie.

On a chromatographié 30 g du produit de l'Exemple 4 ci-dessus sur une colonne de silice MATREX™ SILICA Si (35-78 µm) en utilisant comme éluant un mélange chloroforme : acétone : méthanol : eau - 40 : 20 : 30 : 10. On a évaporé la fraction contenant les monométhacrylates de saccharose sous pression réduite en présence d'inhibiteur. On a obtenu 6,5 g (rdt = 22 %) d'un solide amorphe blanc.

Analyse centésimale : théorique C, 46,80 % ; H 6,34 % expérimentale C, 46,42 % ; H 6,12 %

Exemple 7 :

On a saturé le mélange réactionnel brut obtenu dans l'Exemple 2 ci-dessus en NaCl puis on l'a extrait avec du butanol-2 (8 x 50 ml). On a rassemblé les trois premières extractions, riches en polyméthacrylates, et on les a évaporées, en présence de 3 % d'Ionol pour obtenir une fraction (10 g) enrichie en polyméthacrylates contenant 70 % de polyméthacrylates de saccharose et 30 % de monométhacrylates de saccharose.

Exemple 8

On a dissous 25 g de saccharose (73 mM) dans 75 ml d'eau. On a amène le pH de la solution à 10,5 à l'aide de soude 6N et on l'a maintenu à cette valeur pendant toute la durée de la réaction. On a ajouté goutte à goutte 2,5 ml d'anhydride méthacrylique (18 mM) sous agitation à température ambiante puis on a neutralisé le milieu réactionnel.

On a obtenu les résultats suivants :

- T.T. saccharose : 24 %
- saccharose non réagi : 70 %
- monométhacrylates de saccharose : 25 %
- polyméthacrylates de saccharose : 5 %

Exemple 9

On a dissous 25 g de saccharose (73 mM) dans 75 ml d'eau. On a amené le pH de la solution à 10,5 à l'aide de soude 6N et on l'a maintenu à cette valeur pendant toute la durée de la réaction.

On a ajouté goutte à goutte 7,75 ml (72 mM) de méthacrylate de méthyle tout en agitant pendant 6 h à température ambiante.

On a purifié le mélange par chromatographie (voir Exemple 2 ci-dessus) et on a recueilli 0,8 g de monométhacrylates de saccharose (rdt : 3 %).

Exemple 10 : Acétylation de monométhacrylates de saccharose

On a dissous 2 g (4,9 mM) de monométhacrylates de saccharose dans 20 ml de pyridine et on a ajouté goutte à goutte 10 ml d'anhydride acétique à 0°C. On a maintenu le milieu réactionnel sous agitation pendant 12 h à température ambiante et on l'a évaporé. On a repris le résidu obtenu par 20 ml d'eau et 4 x 20 ml d'éther. On a traité le phase éthérée par une phase aqueuse d'HCl (1N) et de $Na_2CO_3$ (saturé), on a rincé à l'eau, séché puis évaporé.

On a obtenu les produits attendus avec un rendement quantitatif.

Exemple 11 : Purification des monométhacrylates de saccharose par colonne chromatographique sur silice

Les monométhacrylates de saccharose ont été analysés en chromatographie liquide haute performance dans les conditions suivantes : colonne L = 25 cm Ø int. = 4,6 mm, Nucléosil $NH_2$ 5 μm avec un éluant composé de $CH_3CN$ : $H_2O$ -75 : 25, débit 1 ml mn$^{-1}$, détecteur I.R. Les facteurs de capacité caractéristiques sont les suivants :

$$\text{saccharose } k' = \frac{t - to}{to} \times 1,94$$

monométhacrylates de saccharose k'= 0,37

Les monoacrylates et monométhacrylates de saccharose des Exemples ci-dessus ont une pureté, mesurée par HPLC et RMN du proton, supérieure à 95 %.

**Revendications**

1. Procédé de préparation de monomères comportant au moins un groupement acrylate ou méthylacrylate polymérisable d'un saccharide choisi parmi les mono-, di- et trisaccharides, en particulier parmi les mono-, di- et trisaccharides non réducteurs, consistant à faire réagir en milieu aqueux ledit saccharide avec un réactif estérifiant contenant ledit groupement acrylate ou méthylacrylate polymérisable en présence d'une base tout en maintenant le pH du milieu réactionnel à une valeur dans l'intervalle de 7 à 11 pendant toute la durée de la réaction.

2. Procédé selon la revendication 1 dans lequel ledit réactif estérifiant est un halogénure d'acryloyle ou de méthacryloyle

3. Procédé selon la revendication 2 dans lequel lesdits halogénures d'acryloyle ou de méthacryloyle sont des chlorures d'acryloyle ou de méthacryloyle.

4. Procédé selon la revendication 1 dans lequel le réactif estérifiant est un anhydride d'acide tels que l'anhydride acrylique ou l'anhydride méthacrylique.

5. Procédé selon la revendication 1 dans lequel ledit réactif estérifiant est choisi parmi les alkylesters acrylique ou méthacrylique, ledit groupement alkyl ayant de 1 à 3 carbones, tels que l'acrylate de méthyl ou le méthacrylate de méthyl.

6. Procédé selon l'une quelconque des revendications 1 à 5 dans lequel la valeur du pH est maintenue dans l'intervalle de 10 à 11 pendant toute la durée de la réaction.

7. Procédé selon l'une quelconque des revendications 1 à 6 dans lequel le rapport molaire dudit réactif estérifiant sur ledit saccharide de départ est compris entre environ 0,1 et 8.

8. Procédé selon la revendication 7 dans lequel ledit rapport molaire est compris entre environ 0,2 et 2.

9. Procédé selon la revendication 8 dans lequel ledit rapport molaire est compris entre environ 0,2 et 1,5.

10. Procédé selon l'une quelconque des revendications 1 à 9 dans lequel ladite base est une base minérale.

11. Procédé selon la revendication 10 dans lequel ladite base minérale est choisie parmi $Na_2CO_3$, $K_2CO_3$, NaOH et KOH.

12. Procédé selon l'une quelconque des revendications 1 à 11 dans lequel on effectue en outre une phase ultérieure de purification.

13. Procédé selon la revendication 12 dans lequel ladite purification est effectuée par chromatographie ou par cristallisation fractionnée.

14. Procédé selon la revendication 12 dans lequel ladite purification est effectuée par extraction liquide-liquide.

15. Procédé selon la revendication 14 dans lequel ladite extraction liquide-liquide est réalisée en effectuant dans un premier temps une extraction par un solvant organique ou un mélange de solvants organiques suivie dans un deuxième temps d'une extraction par une autre combinaison plus polaire de solvants.

16. Procédé selon l'une quelconque des revendications 1 à 15 dans lequel on effectue une stabilisation des réactifs et/ou des produits recherchés obtenus.

17. Procédé selon la revendication 16 dans lequel ladite stabilisation est effectuée au moyen d'un inhibiteur de polymérisation tel la benzoquinone ou le 2,6-di-tert-butyl-4-méthylphénol.

## Patentansprüche

1. Verfahren zum Herstellen von Monomeren, die zumindest eine polymerisierbare Acrylat- oder Methacrylatgruppe eines Saccharides, ausgewählt aus den Mono-, Di- und Trisacchariden, aufweisen, insbesondere aus den nicht- reduzierenden Mono-, Di- und Trisacchariden, bestehend aus dem Reagierenlassen des Saccharides im wässrigen Medium mit einem veresternden Reagenz, welches die polymerisierbare Acrylat- oder Methacrylatgruppe enthält in Gegenwart einer Base, wobei man während der Gesamtdauer der Reaktion den pH-Wert des Reaktionsmediums auf einem Wert im Intervall von 7 bis 11 hält.

2. Verfahren gemäß Anspruch 1, in dem das veresternde Reagenz ein Acrylsäurechlorid oder Methacrylsäurehalogenid ist.

3. Verfahren gemäß Anspruch 2, in dem die Acrylsäurehalogenide oder Methacrylsäurehalogenide Acrylsäurehalogenide oder Methacrylsäurechloride sind.

4. Verfahren gemäß Anspruch 1, indem das veresternde Reagenz ein Säureanhydrid so wie Acrylsäureanhydrid oder Methacrylsäureanhydrid ist.

5. Verfahren gemäß Anspruch 1, in dem das veresternde Reagenz unter den acrylischen oder methacrylischen Alkylestern gewählt ist, wobei die Alkylgruppe von 1 bis 3 Kohlenstoffatome hat, so wie das Methylacrylat oder Methylmethacrylat.

6. Verfahren gemäß irgendeinem der Ansprüche 1 bis 5, in dem der pH-Wert in dem Intervall von 10 bis 11 während der gesamten Dauer des Verfahrens gehalten wird.

7. Verfahren gemäß irgendeinem der Ansprüche 1 bis 6, in dem das Molverhältnis des veresternden Reagenz zu dem Ausgangssaccharid zwischen ungefähr 0,1 und 8 enthalten ist.

8. Verfahren gemäß Anspruch 7, in dem das Molverhältnis zwischen ungefaähr 0,2 und 2 enthalten ist.

9. Verfahren gemäß Anspruch 8, in dem das Molverhältnis ungefähr zwischen 0,2 und 1,5 enthalten ist.

10. Verfahren gemäß irgendeinem der Ansprüche 1 bis 9, in dem die Base eine Mineralbase ist.

11. Verfahren gemäß Anspruch 10, in dem die Mineralbase unter $Na_2CO_3$, $K_2CO_3$, NaOH und KOH ausgewählt ist.

12. Verfahren gemäß irgendeinem der Ansprüche 1 bis 11, in dem man über dieses eine nachträgliche Reinigungsphase bewirkt.

13. Verfahren gemäß Anspruch 12, in dem die Reinigung durch Chromatographie oder durch fraktionierte Kristallisation bewirkt wird.

14. Verfahren gemäß Anspruch 12, in dem die Reinigung durch Flüssig-Flüssig-Extraktion bewirkt wird.

15. Verfahren gemäß Anspruch 14, in dem die Flüssig-Flüssig-Extraktion verwirklicht wird, indem in einer ersten Zeit eine Extraktion durch ein organisches Lösungsmittel oder eine Mischung von organischen Lösungsmitteln, gefolgt in einer zweiten Zeit von einer Extraktion durch eine andere, polarere Kombination von Lösungsmitteln bewirkt wird.

16. Verfahren gemäß irgendeinem der Ansprüche 1 bis 15, in dem man eine Stabilisierung der Reagenzien und/oder der gesuchten erhaltenen Produkte bewirkt.

17. Verfahren gemäß Anspruch 16, in dem die Stabilisierung mittels eines Polymerisationsinhibitors so wie Benzochinon oder 2,6-di-tert-butyl-4-methylphenol bewirkt wird.

## Claims

1. Process for preparing monomers comprising at least one polymerizable acrylate or methylacrylate group of a saccharide selected from mono- di- and trisaccharides, in particular non-reducing mono-, di, and trisaccharides, consisting of reacting the said saccharide in an aqueous medium with an esterifying reagent containing the said polymerizable acrylate or methacrylate group in the presence of a base, while maintaining the pH of the reaction medium at a value within the interval 7 to 11 during all the duration of the reaction.

2. Process according to claim 1, in which the said esterifying reagent is an acryloyl or methacryloyl halide.

3. Process according to claim 2, in which the said acryloyl or methacryloyl halides are acryloyl or methacryloyl chlorides.

4. Process according to claim 1, in which the said esterifying reagent is an acid anhydride such as acrylic anhydride or methacrylic anhydride.

5. Process according to claim 1, in which the said esterifying reagent is selected from acrylic or methacrylic alkyl esters, the said alkyl group having 1 to 3 carbons, such as methyl acrylate or methyl methacrylate.

6. Process according to any one of claims 1 to 5 in which the value of the pH is maintained within the interval 10 to 11 during all the duration of the reaction.

7. Process according to any one of claims 1 to 6 in which the molar ratio of the said esterifying reagent to the said starting saccharide is between about 0.1 and 8.

8. Process according to claim 7 in which the said molar ratio is between 0.2 and 2.

9. Process according to claim 8 in which the said molar ratio is between 0.2 and 1.5.

10. Process according to any one of claims 1 to 9 in which the said base is an inorganic base.

11. Process according to claim 10 in which the said inorganic base is selected from $Na_2CO_3$, $K_2CO_3$, NaOH and KOH.

12. Process according to any one of claims 1 to 11 in which in addition a subsequent purification phase is carried out.

13. Process according to claim 12 in which the said purification is carried out by chromatography or by fractional crystallization.

14. Process according to claim 12 in which the said purification is carried out by liquid-liquid extraction.

15. Process according to claim 14 in which the said liquid-liquid extraction is performed by first of all carrying out an extraction with an organic solvent or a mixture of organic solvents followed by an extraction with another combination of polar solvents.

16. Process according to any one of claims 1 to 15 in which the reagents and/or the desired products obtained are stabilized.

17. Process according to claim 16 in which the said stabilization is carried out by means of a polymerization inhibitor such as benzoquinone or 2,6-di-tert-butyl-4-methylphenol.